# EUROPEAN PATENT SPECIFICATION

(11) **EP 3 106 135 B1**
(45) Date of publication and mention of the grant of the patent: **25.03.2020**
(21) Application number: 14887618.8
(22) Date of filing: 09.07.2014
(51) Int. Cl.: A61F 2/04, A61F 2/90, A61F 2/07

(54) **STENT FOR PREVENTING REVERSE FLOW**
STENT ZUR VERHINDERUNG VON RÜCKFLUSS
ENDOPROTHÈSE EMPÊCHANT UNE INVERSION DE LA CIRCULATION

(30) Priority: 28.03.2014 KR 20140036728
(43) Date of publication of application: 21.12.2016
(73) Proprietor: S&G Biotech, Inc., Seongnam-si, Gyeonggi-do 462-713 (KR)
(72) Inventor: MOON, Jong Ho, Seoul 07969 (KR); KANG, SungKwon, Yongin-si Gyeonggi-do 448-538 (KR)
(74) Representative: Prüfer & Partner mbB Patentanwälte · Rechtsanwälte
(86) International application number: PCT/KR2014/006137
(87) International publication number: WO 2015/147381

(56) References cited:
- EP-A1- 1 704 834
- WO-A1-2013/115141
- JP-B2- 4 234 588
- KR-A- 20080 078 265
- KR-A- 20110 125 360
- KR-A- 20130 110 421
- KR-B1- 100 691 503
- US-A1- 2013 289 740

## Description

### BACKGROUND OF THE INVENTION

### 1. Field of the Invention

The present invention relates to a stent that is placed in the bile duct connected to the duodenum to facilitate bile secretion into the duodenum therethrough.

### 2. Description of the Related Art

Stents are medical implants that are placed in the human body to render the flows of the body fluids easier or to artificially form fluid passages. Stents can be divided into various types according to their use, material, and shape. Depending on their use, stents can be divided into vascular stents that are placed in narrowed blood vessels to help blood circulation and visceral stents that expand the sites of visceral stenosis or form artificial ducts. Stents can also be divided into metal stents and plastic stents depending on their material. Stents can also be divided into coated stents and uncoated stents depending on whether or not they are coated.

Visceral stents are placed in narrowed fluid ducts to facilitate movement of fluids, form artificial passages between different organs where movement of fluids is required, or form temporary passages through which cyst fluids flow out.

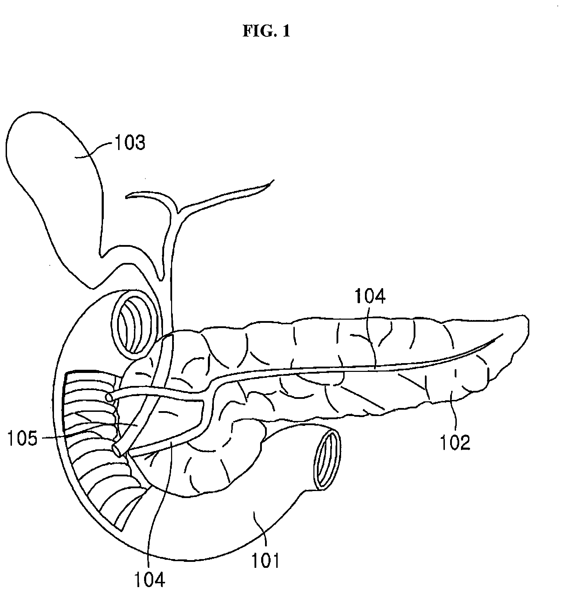

FIG. 1 is an anatomical chart showing major organs of the human body. Referring to FIG. 1, bile stored in the gallbladder 103 is secreted into the duodenum 101 through the bile duct 105 and pancreatic fluid produced in the pancreas 102 is also secreted into the duodenum 101 through the pancreatic duct 104. The bile is produced in the liver and the duodenum is an organ connected to the stomach. In the case where a disease occurs in a duct, such as the bile duct or pancreatic duct, through which a secreted digestive fluid flows, the passage of the duct may be narrowed, impeding secretion of the digestive fluid. In this case, stent placement helps in secreting the digestive fluid.

For example, the incurrence of bile duct cancer may cause narrowing of the bile duct, impeding the secretion of bile into the duodenum. In this case, for a smooth flow of bile, a bypass is formed through which bile travels from the gallbladder to the duodenum or stent placement is performed to expand the narrowed bile duct.

Further, digested food or digestive fluids from the stomach should be prevented from entering the bile duct. In this connection, Korean Patent No. 1179692 discloses a stent including a cover member that functions to prevent digested food from the duodenum from entering the gallbladder. However, the cover member may be inverted and folded toward the interior of the stent. In this case, the cover member loses its function, failing to prevent digested food from the duodenum from entering the gallbladder.

US Patent Application Publication 2013/0289740 discloses a stent that prevents reverse flow by a valve as long as a retrograde pressure is below an antegrade pressure.

### SUMMARY OF THE INVENTION

Therefore, an object of the present invention is to provide a stent that is placed in the bile duct between the gallbladder and the duodenum to facilitate bile secretion therethrough and effectively prevent digested food from the duodenum from entering the gallbladder.

An aspect of the present invention provides a stent that is placed in the bile duct to facilitate bile secretion into the duodenum, including: an elastically deformable body that has a predetermined diameter and length and is formed by interlacing wires in the form of a tube; a film part that surrounds to cover at least a portion of the body, extends a predetermined length from one end of the body, and is made of a flexible material; and an anti-inversion part that is connected to the one end of the body from which the film part extends and prevents the film part from being inverted and folded inwardly toward the body.

The body is divided into a first region uncovered with the film part and a second region covered with the film part.

According to a further embodiment of the present invention, the anti-inversion part may be formed by interlacing a pair of anti-inversion wires extending from the wires of the body such that the anti-inversion wires are moved further away from each other in the direction where the film part extends.

According to an alternative embodiment of the present invention, the anti-inversion wires may extend from the diametrically opposite ends of the body, may be moved closer to each other in the direction where the film part extends, and may meet together.

The stent of the present invention has the following effects.
1. The stent is placed in the bile duct to ensure a passage through which bile flows, allowing the bile to be secreted into the duodenum.
2. The film part covering the body extends in one direction from the body and is made of a flexible material, allowing a fluid to flow smoothly in only one direction. As a result, digested food from the duodenum can be prevented from entering the gallbladder.
3. The body region uncovered with the film part functions to fix the stent within the bile duct and the film part region covering the body effectively prevents the tissue of the bile duct from penetrating between the wires of the body to again occlude the bile duct.
4. The anti-inversion part is arranged in the direction where the film part extends, to prevent the film part from being inverted and folded toward the interior of the stent.

### BRIEF DESCRIPTION OF THE DRAWINGS

These and/or other aspects and advantages of the invention will become apparent and more readily appreciated from the following description of the embodiments, taken in conjunction with the accompanying drawings of which:
FIG. 1 is an anatomical chart showing major organs of the human body;
FIG. 2 illustrates a state in which a stent is placed in the bile duct;
FIGS. 3 and 4 illustrate stents according to embodiments of the present invention;
FIG. 5 shows photographs of a stent according to the present invention;
FIG. 6 explains problems of a conventional stent; and
FIG. 7 illustrates a stent of the present invention in which a film part is prevented from being inverted and folded inwardly.

### DETAILED DESCRIPTION OF THE INVENTION

The present invention provides a stent that is placed in the bile duct to facilitate bile secretion into the duodenum, including: an elastically deformable body that has a predetermined diameter and length and is formed by interlacing wires in the form of a tube; a film part that surrounds to cover a portion of the body, extends a predetermined length from one end of the body, and is made of a flexible material; and an anti-inversion part that is connected to the one end of the body from which the film part extends and prevents the film part from being inverted and folded inwardly toward the body.

The stent of the present invention is placed in the bile duct to facilitate bile secretion from the gallbladder into the duodenum. The film part covering a portion of the stent body extends a predetermined length in one direction from the body and the film part made of a flexible material is foldable so that a fluid is allowed to pass through the stent in only one direction. The anti-inversion part is arranged at one end of the body in the direction where the film part extends, to prevent the film part from being inverted and folded toward the interior of the stent. As a result, the stent can be effectively prevented from losing its antireflux function.

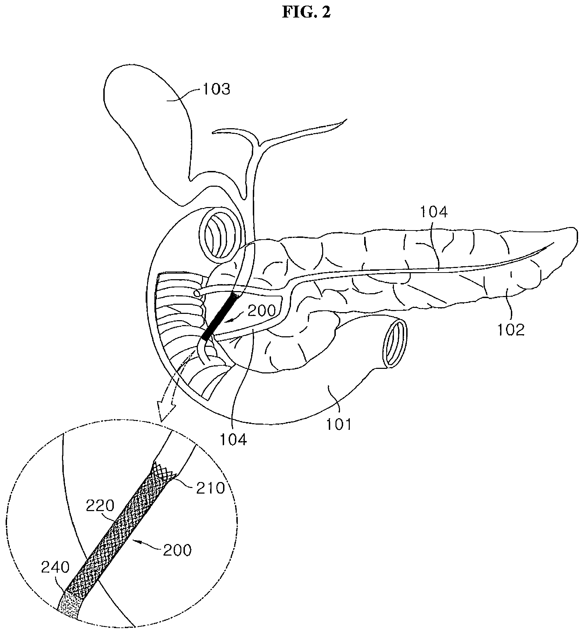

FIG. 2 illustrates a state in which the stent is placed in the bile duct. Referring to FIG. 2, the stent 200 is placed in the bile duct connected to the gallbladder 103 and is connected to the duodenum 101. The stent 200 placed in the bile duct functions to ensure a space through which bile flows. The stent 200 has a first body region 210 uncovered with the film part 240. The first body region 210 functions to fixedly position the stent within the bile duct because the wires of the body support the inner wall of the bile duct. The stent has a second body region 220 covered with the film part. The film part covering the second body region 220 prevents the internal tissue of the bile duct from penetrating into and growing in the body. The film part 240 extends a predetermined length from one end of the second body region 220 and is flexibly placed in the duodenum 101. Accordingly, the film part 240 allows a smooth flow of bile from the bile duct into the duodenum 101 whereas it prevents digested food from the duodenum 101 from flowing toward the bile duct even when folded.

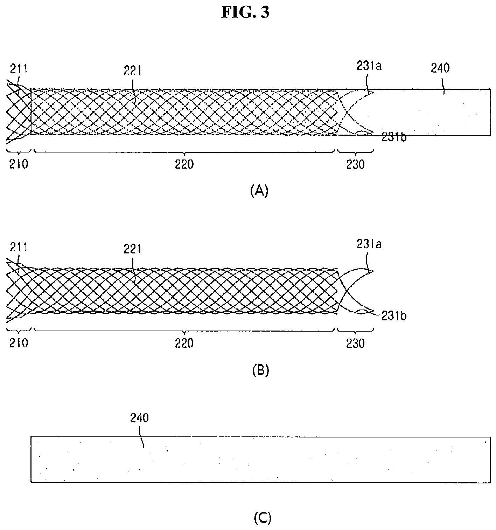

(A) of FIG. 3 illustrates a structure of the stent in which the body is coupled to the film part. (B) and (C) of FIG. 3 illustrate the body and the film part, respectively. Referring to (A) of FIG. 3, the body of the stent is divided into the first region 210 and the second region 220. The first region 210 is not covered with the film part 240 and the second region 220 is covered with the film part 240. The anti-inversion part 230 is arranged at one end of the second body region 220 and the film part 240 extends a predetermined length from the one end of the second body region 220.

Referring to (B) of FIG. 3, the body of the stent is formed by interlacing elastic wires. The first body region 210 is opened wide due to its larger diameter than the second body region 220. Due to this structure, the stent can be fixedly positioned within the bile duct in a more effective manner because the first body region relatively strongly pressurizes the inner wall of the bile duct in a state in which the stent is placed in the bile duct. The wires of the body may be made of an elastic material. Examples of such elastic materials include non-reactive metals, such as stainless steel, metallic materials, such as memory alloys, and elastic polymer resins.

Referring to (C) of FIG. 3, the film part is cylindrical with a predetermined diameter and length such that it can cover the body. The film part is made of a thin flexible material such that its extension uncovering the body is readily foldable and can thus function as a one-way valve. In addition, it is preferred that the film part has a tensile strength sufficient to prevent the tissue of the bile duct from penetrating and growing between the wires. The film part may be made of a non-reactive polymer resin. A preferred material for the film part is PTFE as a fluorinated resin.

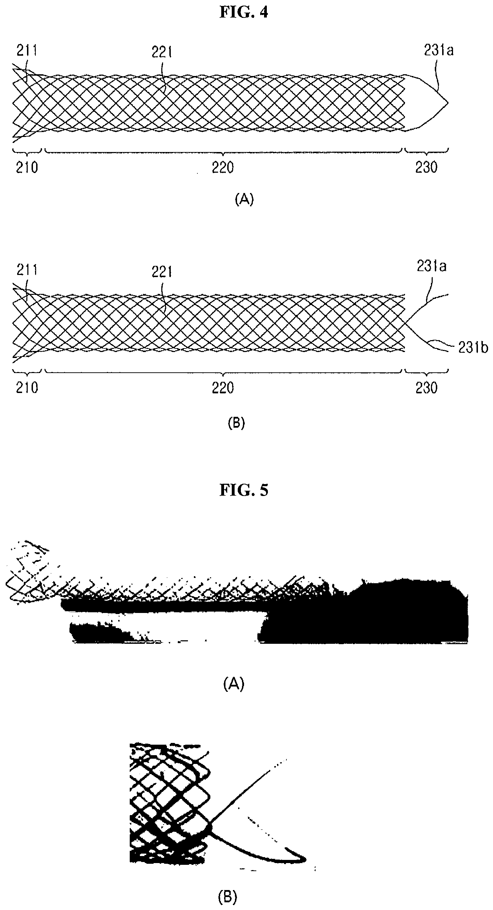

FIG. 4 illustrates the structures of the anti-inversion part of the stent according to the present invention. The anti-inversion part is made of a pair of anti-inversion wires. In FIG. 4, the anti-inversion wires are illustrated as viewed from the front (A) and side (B). Referring to (A) of FIG. 4, the body is made of wires 211 and 221 interlaced with each other and is divided into the first region 210 and the second region 220. The anti-inversion part 230 is arranged at one end of the second body region 220. The anti-inversion wires extend from the diametrically opposite ends of the body, are moved closer to each other in the direction where the film part extends, and meet together. In this figure, only one 231a of the anti-inversion wires is visible. Referring to (B) of FIG. 4, the anti-inversion wires 231a and 231b are connected to the end of the second body region 220 and are moved further away from each other in the direction where the film part extends. In this figure, the sides of the anti-inversion wires are visible.

FIG. 5 shows photographs of a commercially available product of the stent according to the present invention. Referring to FIG. 5, visible are the body formed by interlacing wires, the film part covering a portion of the body and extending from one end of the body, and the anti-inversion part extending from the one end of the body and arranged in the film part.

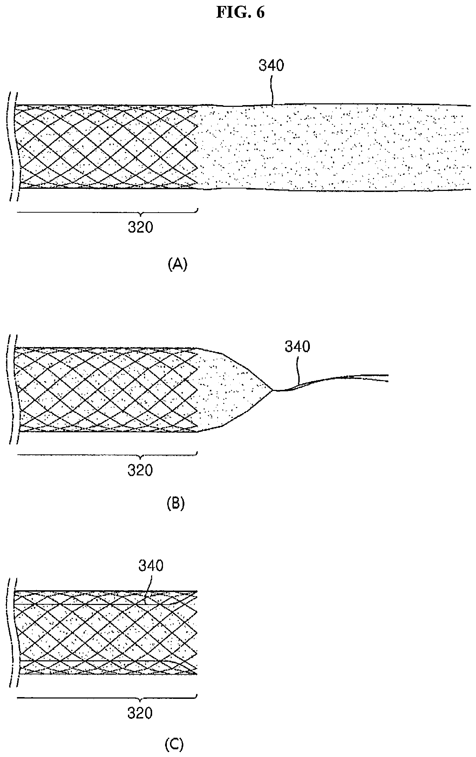

FIG. 6 explains problems of a conventional bile duct insertable stent. Referring to (A) of FIG. 6, the conventional stent includes a film part 340 that covers a body 320 and extends from one end of the body. Referring to (B) of FIG. 6, the film part 340 is made of a flexible material, and as a result, it is bendable and foldable in any portion of its region where the body is not covered. The bent and folded film part clogs the internal passage of the stent and prevents a fluid from entering the body from the film part. When a fluid flows from the body toward the film part, the film part is unfolded. As a result, the film part does not block the flow of the fluid. Referring to (C) of FIG. 6, the film part 340 is freely deformable within the duodenum and can be inverted and folded inwardly toward the body 320. This inversion and folding leads to the formation of an inlet of a passage from the film part toward the body, resulting in a backward flow of digested food and digestive fluids from the duodenum toward the bile duct.

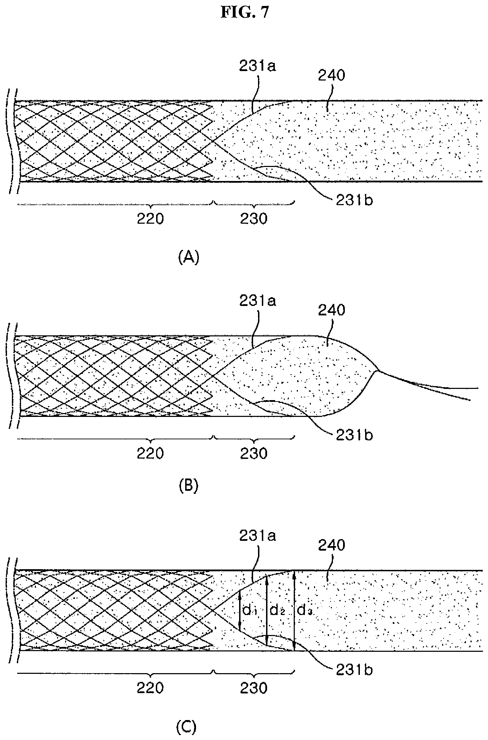

FIG. 7 illustrates the stent of the present invention in which the film part is prevented from being inverted and folded inwardly. Referring to (A) of FIG. 7, the anti-inversion wires 231a and 231b of the anti-inversion part 230 are connected to one end of the second region 220. The anti-inversion part 230 prevents the film part from being folded inwardly above a predetermined length. Referring to (B) of FIG. 7, the film part 240 is foldable in its region where the body is not covered. In this case, the film part clogs the inlet of the passage through which fluids flow from the duodenum toward the bile duct. Referring to (C) of FIG. 7, the anti-inversion wires 231a and 231b of the anti-inversion part 230 are moved further away from each other with increasing distance outward from the center of the end of the second region 220. That is, the distance between the anti-inversion wires 231a and 231b decreases in the order of d3, d2, and d1 in the direction opposite to the direction where the film part extends. The formation of the anti-inversion part at one end of the second region of the body makes it difficult for the film part to be inverted and folded inwardly toward the body.

Although the present invention has been described herein with reference to the foregoing embodiments, those skilled in the art will appreciate that various changes and modifications are possible, without departing from the essential features of the present invention. Therefore, the embodiments do not serve to limit the invention and are set forth for illustrative purposes. The scope of the invention is defined by the appended claims and all changes or modifications made within the scope of the claims should be construed as falling within the scope of the invention.

## Claims

1. A stent comprising:
an elastically deformable body (210, 220) that has a predetermined diameter and length and is formed by interlacing wires in the form of a tube;
a film part (240) that surrounds to cover at least a portion of the body, extends a predetermined length from one end of the body, and is made of a flexible material; and
an anti-inversion part (230) that is connected to the one end of the body from which the film part extends, **characterized in that**
the stent is configured to be placed in the bile duct to facilitate bile secretion into the duodenum
the anti-inversion part is configured to prevent the film part from being inverted and folded inwardly toward the body to prevent digested food from the duodenum from entering the gallbladder, and
the body is divided into a first region (210) uncovered with the film part and a second region (220) covered with the film part.

2. The stent according to claim 1, wherein the anti-inversion part is formed by interlacing a pair of anti-inversion wires extending from the wires of the body such that the anti-inversion wires are moved further away from each other in the direction where the film part extends.

3. The stent according to claim 2, wherein the anti-inversion wires extend from the diametrically opposite ends of the body, are moved closer to each other in the direction where the film part extends, and meet together.

## Patentansprüche

1. Ein Stent, umfassend:
einen elastisch verformbaren Körper (210, 220), der einen vorbestimmten Durchmesser und eine vorbestimmte Länge aufweist und durch Verflechtung von Drähten in der Form eines Rohrs gebildet ist,
einen Folienabschnitt (240), der zumindest einen Teil des Körpers umgibt, um diesen abzudecken, sich über eine vorbestimmte Länge von einem Ende des Körpers erstreckt und aus einem flexiblen Material ist, und
einen Anti-Inversionsabschnitt (230), der mit dem einen Ende des Körpers, von dem sich der Folienabschnitt erstreckt, verbunden ist, **dadurch gekennzeichnet, dass**
der Stent dazu ausgebildet ist, im Gallengang platziert zu werden, um die Gallenflüssigkeitssekretion in den Zwölffingerdarm zu ermöglichen,
der Anti-Inversionsabschnitt dazu ausgebildet ist, den Folienabschnitt darin zu hindern, zum Körper hin umgekehrt und nach innen gefaltet zu werden, um zu verhindern, dass verdaute Nahrung vom Zwölffingerdarm in die Gallenblase eintritt, und
der Körper in einen ersten Bereich (210), der nicht mit dem Folienabschnitt bedeckt ist, und einen zweiten Bereich (220), der mit dem Folienabschnitt bedeckt ist, unterteilt ist.

2. Der Stent nach Anspruch 1, wobei der Anti-Inversionsabschnitt durch Verflechtung eines Paars von Anti-Inversionsdrähten gebildet wird, die sich von den Drähten des Körpers erstrecken, so dass die Anti-Inversionsdrähte in der Richtung, in die sich der Folienabschnitt erstreckt, weiter voneinander weg bewegt werden.

3. Der Stent nach Anspruch 2, wobei sich die Anti-Inversionsdrähte von den diametral gegenüberliegenden Enden des Körpers erstrecken, in der Richtung, in die sich der Folienabschnitt erstreckt, näher zu einander bewegt werden und sich treffen.

## Revendications

1. Endoprothèse comprenant :
un corps déformable élastiquement (210, 220) qui présente un diamètre et une longueur prédéterminés et est formé par entrelacement de fils métalliques sous la forme d'un tube ;
une partie formant film (240) qui entoure au moins une portion du corps de manière à la couvrir, s'étend sur une longueur prédéterminée à partir d'une extrémité du corps et est constituée d'un matériau souple ; et
une partie anti-inversion (230) qui est reliée à l'extrémité du corps à partir de laquelle s'étend la partie formant film, **caractérisée en ce que**
l'endoprothèse est configurée pour être placée dans le canal cholédoque pour faciliter la sécrétion de bile dans le duodénum
la partie anti-inversion est configurée pour empêcher la partie formant film d'être inversée et pliée vers l'intérieur en direction du corps pour empêcher que des aliments digérés provenant du duodénum pénètrent dans la vésicule biliaire, et
le corps est divisé en une première région (210) non couverte par la partie formant film et une seconde région (220) couverte par la partie formant film.

2. Endoprothèse selon la revendication 1, dans laquelle la partie anti-inversion est formée par entrelacement d'une paire de fils métalliques anti-inversion s'étendant à partir des fils métalliques du corps de sorte que les fils métalliques anti-inversion s'éloignent davantage l'un de l'autre dans la direction dans laquelle s'étend la partie formant film.

3. Endoprothèse selon la revendication 2, dans laquelle les fils métalliques anti-inversion s'étendent à partir des extrémités diamétralement opposées du corps, se rapprochent l'un de l'autre dans la direction dans laquelle s'étend la partie formant film et se rejoignent.
